# FASCICULE DE BREVET EUROPEEN

(11) **EP 1 007 532 B1**
(45) Date de publication et mention de la délivrance du brevet: **23.04.2003**
(21) Numéro de dépôt: 98941566.6
(22) Date de dépôt: 07.08.1998
(51) Int. Cl.: C07H 21/00, C12N 15/11, C12Q 1/68

(54) **OLIGONUCLEOTIDES PERMETTANT L'IDENTIFICATION DE PRECURSEURS D'HORMONES POLYPEPTIDIQUES AMIDEES**
OLIGONUKLEOTIDE ZUR IDENTIFIZIERUNG VON AMIDIERTEN POLYPEPTID-HORMONVORLÄUFERN
OLIGONUCLEOTIDES FOR IDENTIFYING PRECURSORS OF AMIDATED POLYPEPTIDE HORMONES

(30) Priorité: 26.08.1997 FR 9710643
(43) Date de publication de la demande: 14.06.2000
(73) Titulaire: SOCIETE DE CONSEILS DE RECHERCHES ET D'APPLICATIONS SCIENTIFIQUES (S.C.R.A.S.), 75016 Paris (FR); Centre National De La Recherche Scientifique, 75794 Paris Cedex 16 (FR)
(72) Inventeur: MARTINEZ, Jean, F-34570 Saussan (FR); GOZE, Catherine, Résidence Château d'Alco, F-34080 Montpellier (FR)
(74) Mandataire: Bourgouin, André
(86) Numéro de dépôt international: FR9801767
(87) Numéro de publication internationale: WO99010361

(56) Documents cités:
- A.G.KATOPODIS ET AL.: "Functional and Structural Characterization of Peptidylamidoglycolate Lyase, the Enzyme Catalyzing the Second Step in Peptide Amidation." BIOCHEMISTRY., vol. 30, no. 25, 25 juin 1991, pages 6189-6194, XP002074069 EASTON, PA US cité dans la demande
- S.A.NARANG: "dna synthesis" TETRAHEDRON., vol. 39, no. 1, 1983, pages 3-22, XP002074070 OXFORD GB cité dans la demande

## Description

La présente invention a pour objet de nouveaux oligonucléotides et leur application comme sondes pour l'identification des ARNm codant pour les précurseurs d'hormones polypeptidiques amidées et, ainsi, l'identification de nouvelles hormones polypeptidiques amidées. L'invention concerne donc des oligonucléotides dont la séquence nucléotidique est décrite ci-après et une méthode d'identification des précurseurs d'hormones amidées.

Les hormones polypeptidiques amidées sont synthétisées sous forme d'un précurseur qui subit une maturation. Cette maturation consiste en une réaction d'amidation.

La réaction d'amidation de l'extrémité C-terminale est une réaction caractéristique des hormones polypeptidiques amidées. Cette réaction, qui intervient sur le précurseur d'une ou plusieurs hormones, permet la maturation de l'hormone et assure également sa biostabilité dans le milieu physiologique : le groupement amide formé est moins vulnérable que la fonction acide libre. L'hormone est alors plus résistante aux carboxypeptidases, elle reste active plus longtemps dans la cellule et garde une affinité optimale avec son site récepteur.

L'amidation a été largement décrite (" Peptide amidation ", Alan F. Bradbury et Derek G. Smyth, TIBS 16 : 112-115, March 1991 et " Functional and structural characterization of peptidylamidoglycolate lyase, the enzym catalyzing the second step in peptide amidation ", A.G. Katopodis, D.S. Ping, C.E. Smith and S.W. May, Biochemistry, 30(25) : 6189-6194, June 1991), son mécanisme est le suivant :
1- clivage de la chaîne polypeptidique précurseur de l'hormone par une endoprotéase au niveau de deux acides aminés basiques qui sont l'arginine et/ou la lysine,
2 - ensuite, se produisent deux clivages par la carboxypeptidase qui conduisent à l'intermédiaire glycine étendu,
3 - l'enzyme PAM (Peptidyl-glycine-α-Amidating Monooxygenase) comprend deux activités enzymatiques distinctes : dans un premier temps, elle convertit l'intermédiaire glycine étendu en dérivé α-hydroxyglycine, la sous unité de l'enzyme PAM qui intervient est la PHM (Peptidyl-glycine-α-Hydrolylating Monooxygenase). Le dérivé obtenu sert de substrat à la deuxième sous unité de la PAM (notée PAL : Peptydil-α-hydroxyglycine α-Amidating Lyase) qui fixe la fonction amine de la glycine sur l'acide aminé immédiatement adjacent du coté N-terminal et libère le glyoxylate.

Cette réaction impose la présence d'un site de reconnaissance sur le précurseur de ou des hormones, site qui comporte toujours la séquence : glycine et deux acides aminés basiques (arginine ou lysine) (cf. A.G. Katopodis et coll., Biochemistry, **30**(25), 6189-6194. June 1991, et références citées).

Les hormones polypeptidiques amidées qui sont destinées à être sécrétées hors du réticulum endoplasmique sont connues pour comporter une séquence consensus signal d'une quinzaine à une trentaine d'acides aminés, cette séquence est présente à l'extrémité N-terminale de la chaîne polypeptidique. Elle est coupée plus tard par une enzyme signal peptidase, de sorte qu'on ne la retrouve plus dans la protéine une fois sécrétée (cf. F. Cuttitta, *The Anatomical Record,* **236,** 87-93 (1993) et références citées).

A l'heure actuelle, la découverte d'une nouvelle protéine n'est pas une chose aisée. L'isolement et la purification des protéines peuvent se faire par des techniques diverses : précipitation au point isoélectrique, extraction sélective par certains solvants, puis purification par cristallisation, distribution à contre courant, chromatographie d'adsorption, de partage ou par échange d'ions, électrophorèse... Cependant ces techniques sous-entendent la connaissance des propriétés de la protéine à isoler. Par ailleurs, lorsqu'on dispose d'un échantillon pur d'une nouvelle protéine intéressante sur le plan thérapeutique, il reste encore de nombreuses étapes avant de disposer d'un microorganisme génétiquement modifié capable de la synthétiser.

La méthode proposée par la présente invention offre l'avantage, par l'utilisation d'une particularité de la séquence peptidique du précurseur de toutes hormones amidées connues à ce jour, de permettre la mise en évidence simultanée de plusieurs nouvelles hormones de cette catégorie. Cette recherche se fait par l'identification directe de la séquence nucléotidique codant pour lesdits précurseurs dans des banques d'ADNc préparées à partir de tissus dans lesquels les précurseurs de ces hormones sont susceptibles d'être synthétisés.

La recherche par cette méthode est beaucoup moins contraignante que les techniques classiques de biochimie citées précédemment car
- elle peut conduire à isoler, suivant le même principe, plusieurs précurseurs distincts présents dans le même tissu ;
- elle permet de mettre en évidence, dans les mêmes conditions techniques, des précurseurs correspondant à des hormones ayant des propriétés biochimiques et biologiques très différentes ;
- elle autorise l'identification concomitante de toutes les hormones peptidiques pouvant être contenues dans le même précurseur.

De ce fait, cette invention permet un gain de temps et d'argent non négligeable dans un secteur où les dépenses de Recherche et Développement représentent une proportion très importante du chiffre d'affaires.

La présente invention permettra également l'étude pharmacologique de substances actives ayant un rôle physiologique fondamental dans l'organisme des mammifères : les hormones et plus particulièrement les neurohormones polypeptidiques amidées. Disposant en premier lieu des ADNc correspondant aux substances actives, il sera alors possible d'introduire par génie génétique le vecteur cloné pour mener la synthèse des hormones ayant une application thérapeutique par des micro-organismes.

L'invention a d'abord pour objet un oligonucléotide OX simple brin pouvant s'hybrider dans des conditions douces avec un oligonucléotide OY de séquence Y1-Y2-Y3-Y4-Y5, dans laquelle Y1 représente une séquence nucléotidique de 1 à 12 nucléotides ou Y1 est supprimé, Y2 représente un trinucléotide codant pour Gly, Y3 et Y4 représentent indépendamment un trinucléotide codant pour Arg ou Lys, et Y5 représente une séquence nucléotidique de 1 à 21 nucléotides ou Y5 est supprimé.

On entend par nucléotide une unité monomère de l'ARN ou de l'ADN ayant la structure chimique d'un ester phosphorique de nucléoside. Un nucléoside résulte de la liaison d'une base purique (purine, adénine, guanine ou analogues) ou d'une base pyrimidique (pyrimidine, cytosine, uracile ou analogues) avec le ribose ou le désoxyribose. Un oligonucléotide est un polymère de nucléotides désignant une séquence amorce, une sonde ou un fragment d'ARN ou d'ADN.

Les oligonucléotides cités peuvent être obtenus par synthèse, il existe une méthode automatisée de référence qui est décrite dans les publications suivantes : "DNA synthesis" de S.A. Narang, Tetrahedron, 39, 3 (1983) et "Synthesis and use of synthetic oligonucleotides" de K. Itakura, J.J. Rossi et R.B. Wallace, Annu. Rev. Biochem., 53, 323 (1984).

De préférence, OX peut s'hybrider dans des conditions stringentes avec OY.

De façon plus préférentielle, OX peut s'hybrider avec un oligonucléotide OY de séquence Y2-Y3-Y4-Y5.

De façon encore plus préférentiellement, OX peut s'hybrider avec un oligonucléotide OY de séquence Y1-Y2-Y3-Y4 ou Y2-Y3-Y4.

Particulièrement, OX peut s'hybrider avec un oligonucléotide OY tel que Y5 représente une séquence nucléotidique Y6-Y7-Y8-Y9 dans laquelle Y6 représente un trinucléotide codant pour Ser, Thr, ou Tyr, Y7 représente un trinucléotide codant pour un acide aminé quelconque, Y8 représente un trinucléotide codant pour Glu ou Asp et Y9 représente une séquence nucléotidique comprenant de 1 à 12 nucléotides. Plus particulièrement, OX peut s'hybrider avec un oligonucléotide OY tel que Y1 et Y9 sont supprimés.

Tout particulièrement, OX peut s'hybrider avec un oligonucléotide OY dans lequel Y2 représente un trinucléotide codant pour Gly, Y3 représente un trinucléotide codant pour Lys, Y4 un trinucléotide codant pour Arg et Y5 une séquence de 3 trinucléotides codant pour Ser-Ala-Glu.

Cette séquence a été déterminée grâce à une étude statistique sur 27 sites d'amidation connus et a conduit à définir sur 6 positions un motif donné d'acides aminés : Gly-Lys-Arg-Ser-Ala-Glu.

En raison de la dégénérescence du code génétique et du nombre élevé de codons correspondant à la Gly (4 codons), à l'Arg (6 codons) et à la Ser (6 codons), la séquence de l'oligonucléotide a été construite à l'aide de deux procédés permettant de prendre en compte cette dégénérescence :
- utilisation à certaines positions de l'inosine, nucléotide dont la base azotée, l'hypoxanthine, s'apparie indifféremment avec les 4 bases azotées constitutives de l'ADN,
- variation, à certaines positions, de la nature de la base azotée incorporée générant ainsi un nombre de combinaison d'oligonucléotides proportionnel au nombre de bases différentes introduites.

La présente invention a également pour objet un oligonucléotide OY comportant de 9 à 42 nucléotides de séquence Y1-Y2-Y3-Y4-Y5, dans laquelle Y1 représente une séquence nucléotidique de 1 à 12 nucléotides ou Y1 est supprimé, Y2 représente un trinucléotide codant pour Gly, Y3 et Y4 représentent indépendamment un trinucléotide codant pour Arg ou Lys, et Y5 représente une séquence nucléotidique de 1 à 21 nucléotides ou Y5 est supprimé.

De préférence, l'invention a pour objet un oligonucléotide OY tel que Y1 est supprimé ou tel que Y5 est supprimé.

L'invention a particulièrement pour objet un oligonucléotide OY tel que Y5 représente une séquence nucléotidique Y6-Y7-Y8-Y9, dans laquelle Y6 représente un trinucléotide codant pour Ser, Thr ou Tyr, Y7 représente un trinucléotide codant pour un acide aminé quelconque, Y8 représente un trinucléotide codant pour Glu ou Asp et Y9 représente une séquence nucléotidique comprenant 1 à 12 nucléotides.

L'invention a plus particulièrement pour objet un oligonucléotide OY tel que Y1 et Y9 sont supprimés.

L'invention a tout particulièrement pour objet un oligonucléotide OY, caractérisé en ce que Y1 est supprimé, Y2 représente un trinucléotide codant pour Gly, Y3 représente un trinucléotide codant pour Lys, Y4 un trinucléotide codant pour Arg et Y5 une séquence de trois trinucléotides codant pour Ser-Ala-Glu.

La présente invention concerne aussi un oligonucléotide simple brin OZ, caractérisé en ce qu'il comprend de 15 à 39 nucléotides et est capable de s'hybrider avec une séquence consensus signal caractéristique des hormones polypeptidiques amidées, ladite séquence ayant pour formule Z1-Z2-Z3-Z4-Z5-Z6-Z7 dans laquelle Z1 représente une séquence nucléotidique de 1 à 12 nucléotides ou Z1 est supprimé, Z2 et Z3 représentent deux trinucléotides codant pour Leu, Z4 et Z5 représentent deux trinucléotides codant pour deux acides aminés quelconques, Z6 représente un trinucléotide codant pour Leu, et Z7 représente une séquence nucléotidique de 1 à 12 nucléotides ou Z7 est supprimé.

Dans cette invention, on entendra par hormone, les hormones polypeptidiques amidées du système endocrinien, et plus particulièrement les neurohormones.

La séquence consensus signal est une séquence portée par les précurseurs des protéines qui sont sécrétées par les cellules après leur maturation.

La présente invention concerne enfin un ensemble d'oligonucléotides OX ou OZ tel qu'il constitue une librairie combinatoire.

Dans l'invention décrite, on entend par librairie combinatoire un ensemble d'oligonucléotides synthétisés en prenant pour modèle une séquence nucléotidique codant pour une séquence d'acides aminés dont certains peuvent être variables. Du fait de la dégénérescence du code génétique, on obtiendra un ensemble d'oligonucléotides différents.

Un autre objet de l'invention est une méthode d'identification du précurseur d'un peptide ayant une extrémité C-terminale amidée, caractérisée par les étapes successives suivantes :
1 - obtention d'une banque d'ADN;
2 - hybridation d'un ou plusieurs oligonucléotides OX avec ladite banque d'ADN;
3 - identification de la ou les séquences d'ADN de ladite banque qui s'hybride avec un oligonucléotide OX;
4 - identification dans cette ou ces séquences d'un ou plusieurs peptides avec une possible extrémité C-terminal amidée.

On préférera une méthode telle que la banque d'ADN est une banque d'ADNc.

L'ADN complémentaire (ADNc) est une chaîne nucléotidique dont la séquence est complémentaire de celle d'un ARNm, la réaction conduisant aux ADNc monocaténaire est catalysée par la transcriptase inverse. L'ADNc bicaténaire peut être obtenu par action de l'ADN polymérase, il est ensuite inséré à l'aide d'une ligase dans un plasmide ou un vecteur dérivé du bactériophage λ.

Une banque d'ADNc regroupe les ADNc correspondant aux ARNm cytoplasmiques extrait d'une cellule donnée. La banque est dite complète lorsqu'elle comprend au moins un clone bactérien pour chaque ARNm de départ.

L'hybridation a lieu lorsque deux oligonucléotides ont des séquences nucléotidiques substantiellement complémentaires, ils peuvent s'associer sur leur longueur par établissement de liaisons hydrogène entre bases complémentaires.

On préférera particulièrement une méthode telle que l'oligonucléotide OX est détectable à l'aide d'un agent de marquage, tel que le ³²P ou la digoxygénine.

Les agents de marquage radioactif des nucléotides les plus couramment utilisés sont les éléments qui émettent des rayons β, par exemple, ³H, ¹²C, ³²P, ³³P et ³⁵S.

Le marquage de l'oligonucléotide se fait par l'addition à son extrémité 5' d'un groupement phosphate apporté par le (γ-³²P)-ATP, cette réaction est catalysée par l'enzyme T4-polynucléotide kinase. Le marquage par la digoxygénine est immunoenzymatique, la digoxygénine est associée à une base azotée et incorporée à l'oligonucléotide. Sa présence est révélée par l'utilisation d'un anticorps dirigé contre la digoxygénine et couplé à une phosphatase alcaline. La révélation utilise la couleur développée par un substrat hydrolysé par la phosphatase alcaline.

D'autres techniques de marquage peuvent être employées : oligonucléotides modifiés chimiquement pour qu'ils contiennent un agent de complexation des métaux (on utilise souvent des complexes du lanthanide), un groupe contenant de la biotine ou ester d'acridine, un composé fluorescent (fluorescine, rhodamine, Texas red) ou autre.

On préférera tout particulièrement une méthode d'identification de précurseur d'hormone polypeptidique amidée telle que l'étape d'hybridation utilise une librairie combinatoire d'oligonucléotides OX.

L'invention a encore pour objet une méthode d'identification du précurseur d'un peptide ayant une extrémité C-terminale amidée, qui comprend les étapes suivantes :
1 - obtention d'une banque d'ADN;
2 - utilisation de la technique PCR pour amplifier le fragment d'intérêt à l'aide d'un ensemble d'oligonucléotides OX et un autre ensemble d'oligonucléotides OZ;
3 - identification de la séquence d'ADN de ladite banque qui s'hybride avec l'oligonucléotide OX et qui atét amplifiée par la réction de PCR;
4 - identification dans cette séquence d'un ou plusieurs peptides avec une possible extrémité C-terminale amidée.

On entend par fragment d'intérêt la séquence d'ADNc codant pour le précurseur d'une ou plusieurs hormones polypeptidiques amidées.

La réaction d'amplification de l'ADN par PCR (Polymerase Chain Reaction) nécessite une préparation d'ADN dénaturé par chauffage à 95°C. Ensuite, cette préparation est appariée à un excès de deux oligonucléotides complémentaires aux brins opposés de l'ADN, de part et d'autre de la séquence à amplifier. Chaque oligonucléotide sert ensuite d'amorce à une DNA polymérase (extraite de bactéries thermophiles du type Thermus aquatitus : Taq polymérase) pour la copie de chacun des brins de l'ADN. Ce cycle peut être répété, de manière automatisée, par dénaturations-renaturations successives.

Il existe de nombreuses références détaillant les protocoles du PCR : Brevets US n° 4.683.192, 4.683.202, 4.800.159 et 4.965.188, "PCR technology : principles and applications for DNA amplification", H. Erlich, ed. Stockton Press, New York (1989) et "PCR protocols : a guide to methods and applications", Innis et al., eds. Academic Press, San Diego, California (1990).

De préférence, ladite banque d'ADN est une banque d'ADNc.

Plus préférentiellement, ledit oligonucléotide OX est détectable à l'aide d'un agent de marquage tel que le ³²P ou la digoxygénine.

On préférera particulièrement une méthode d'identification de précurseur d'hormone polypeptidique amidée telle que l'étape d'amplification utilise une librairie combinatoire d'oligonucléotides OX et une autre librairie combinatoire d'oligonucléotides OZ.

Un autre objet de l'invention est une méthode d'identification du précurseur d'un peptide ayant une extrémité C-terminale amidée, qui comprend les étapes suivantes :
1 - obtention d'une banque d'ADN;
2 - utilisation de la technique PCR pour amplifier le fragment d'intérêt à l'aide d'un ensemble d'oligonucléotides OX;
3 - identification de la séquence d'ADN de ladite banque qui s'hybride avec l'oligonucléotide OX et qui atét amplifiée par la réction de PCR;
4 - identification dans cette séquence d'un ou plusieurs peptides avec une possible extrémité C-terminale amidée.

Le but de cette méthode est de caractériser les séquences nucléotidiques codant pour des précurseurs présentant plus d'un site d'amidation.

De préférence, ladite banque d'ADN est une banque d'ADNc.

Plus préférentiellement, ledit oligonucléotide OX est détectable à l'aide d'un agent de marquage tel que le ³²P ou la digoxygénine.

On préférera particulièrement une méthode d'identification de précurseur d'hormone polypeptidique amidée telle que l'étape d'amplification utilise une librairie combinatoire d'oligonucléotides OX.

Une autre méthode proposée par la présente invention pour l'identification du précurseur d'un polypeptide ayant une extrémité C-terminale amidée, est caractérisée par les étapes suivantes :
1 - obtention d'une banque d'ADN;
2 - utilisation de la technique PCR pour amplifier le fragment d'intérêt à l'aide d'un oligonucléotide OX et un autre oligonucléotide simple brin capable de s'hybrider en conditions douces ou stringentes avec une séquence consensus universelle contenue dans la séquence du vecteur plasmidique dans lequel sont clonés les ADN de la dite banque d'ADN, tels que les primers T3, T7, KS, SK, M13, Reverse;
3 - identification de la séquence d'ADN de ladite banque qui s'hybride avec un oligonucléotide OX;
4 - identification dans cette séquence d'un ou plusieurs peptides avec une possible extrémité C-terminale amidée.

La séquence consensus universelle est une séquence portée par le vecteur dans lequel est cloné l'ADN de la banque. Cette séquence peut servir d'amorce pour le séquençage. Les séquences nucléotidiques de ces primers sont disponibles dans : Sambrook, J., Fritsch, E.F., Maniatis, T., "*Molecular cloning, a laboratory manual*", 2nd edition, **1989,** Colel Spring Harbor Laboratory Press.

La réaction du PCR nécessite que deux oligonucléotides se fixent sur l'ADNc cloné dans un vecteur pour que son amplification ait lieu. Dans le cas où l'on ne connaît qu'une seule séquence propre au fragment d'ADN à amplifier, une solution pour contourner ce problème est d'utiliser un oligonucléotide qui pourra s'hybrider avec une séquence nucléotidique propre au vecteur dans lequel a été cloné l'ADNc, telle qu'une séquence consensus universelle.

De préférence, ladite banque d'ADN est une banque d'ADNc.

On préférera un oligonucléotide OY détectable à l'aide d'un agent de marquage tel que le ³²P ou la digoxygénine.

On préférera plus particulièrement une étape d'amplification utilisant une librairie combinatoire d'oligonucléotides OX.

### EXEMPLE :

La méthode décrite par l'invention a été validée par son application sur une hormone déjà isolée. La neurohormone choisie est la cholécystokinine (CCK) qui est le neuromédiateur quantitativement le plus important représenté dans le cerveau.

### 1.1. Préparation de la matrice d'ADN servant aux réactions de PCR à partir d'une banque commerciale Lambda Zapp II (Rat Brain cDNA Library Vector, réf. 936 501) de STRATAGENE (Lafolla, U.S.A.).

Cette banque d'ADNc Stratagene contient le clonage des ADNc de cellules de cerveau de rat.

### 1.1.1. Libération des ADNc clonés sous forme de phagemides Bluescript (Stratagene, Lajolla, U.S.A.).

Elle se fait en suivant le protocole suivant : on met en contact pendant 15 minutes à 37°C, 250 µl de la banque d'ADNc à 2.10⁸ PFU/ml, 200 µl de bactéries XL₁ blue (génotype : recA1 endA1 gyrA96 thi-1 hsdR17 supE44 relA1 lac [F' proAB lacI^{q}ZΔM15 Tn10 (Tet^{r})]^{c} - cf. Bullock, Fernandez, Short, *Biotechniques,* **5,** 376-379 (1987) - densité optique à 600 nm : D.O. = 2,5) et 1 µl de phage ExAssist™ (cf. Hay, B., Short, J., *Strategies*, **5,** 16-18 (1992)) à 10¹⁰ PFU/ml. Puis l'ensemble est incubé sur 50 ml de milieu LB (composition : pour 1 litre d'eau physiologique stérile, on mélange 10 g de NaCl, 5 g d'extrait de levure et 10 g de Bactotryptone) pendant 3 heures sous agitation à 37°C. Le bouillon de culture est centrifugé puis le surnageant est inactivé par chauffage à 70°C pendant 20 minutes.

### 1.1.2. Obtention des ADNc sous forme d'une banque de plasmides double brin.

Cette étape nécessite 15 minutes d'incubation à 37°C de 100 µl du surnageant inactivé et 200 µl de bactéries SOLR™ (génotype : e14⁻(McrA⁻) Δ(mcrCB-hsdSMR-mrr)171 sbcC recB recJ uvrC umuC::Tn5 (Kan^{r}) lac gyrA96 relA1 thi-1 endA1 λ^{R} [F' proAB lacI^{q}ZΔM15]^{c} Su⁻ (nonsuppressing) - cf. Hay, B., Short, J.M., *Strategies*, **5**(1), 16-18 (1992) - D.O. = 1 à 600 nm). Après addition de 50 µl d'ampicilline (à 100 mg/ml) et de 50 ml de milieu LB, le tout est mis à incuber à 37°C sous agitation pendant une nuit. Les plasmides sont préparés à partir de 50 ml de culture avec le protocole et les colonnes QIAGEN Plasmid Midi Kit de QIAGEN des colonnes QIAGEN contiennent une résine d'échange anionique possédant à sa surface des groupes diéthylaminoéthanol chargés positivement, lesquels interagissent avec les phosphates du squelette de l'ADN). On a ainsi obtenu une solution d'ADN à 1,37 µg/µl.

### 1.2. Amplification d'une portion du précurseur de la CCK à partir de la banque de plasmides ainsi préparée.

### 1.2.1. Etablissement des séquences des deux oligonucléotides nécessaires à la réaction de PCR.

L'un de ces deux nucléotides contiendra la séquence complémentaire à celle codant pour le site d'amidation de la CCK, ce site est connu et a pour séquence Gly-Arg-Arg-Ser-Ala-Glu. Cet oligonucléotide, que l'on nommera *oligo CCK amid,* a pour séquence nucléotidique :

5’ CTCAGCACTGCGCCGGCC 3’

Le second oligonucléotide, noté *oligo CCK 5',* correspond à la séquence consensus signal :

5' GTGTGTCTGTGCGTGGTG 3'

La taille du produit d'amplification attendu est de 315 paires de bases, c'est la distance existant entre les séquences correspondant à ces deux oligonucléotides sur la séquence précurseur de la CCK.

### 1.2.2. Réaction de PCR.

On prépare une dilution D₁ contenant 1 µl d'enzyme Taq polymérase Goldstar 5 U/µl (cf. Reynier, P., Pellissier, J.F., Harle, J.R., Malthiéry, Y., *Biochemical and Biophysical Research Communications*, **205**(1), 375-380 (1994)), 1 µl d'un tampon 10 fois concentré en Taq polymérase standard et 8 µl d'eau.

Puis on mélange 1 µl d'*oligo CCK 5'* à 250 ng/µl, 1 µl d'*oligo CCK amid* à 250 ng/µl, 1 µl dNTP à 10 mM chacun, 1 µl d'ADN banque ADNc à 250 ng/µl, 5 µl de tampon 10 fois concentré de l'enzyme Taq polymérase, 2 µl MgCl₂ à 25 mM, 1 µl de la dilution D₁ et 37 µl d'eau.

Les conditions d'amplification sont les suivantes : on effectue d'abord un traitement thermique de 5 minutes à 95°C, puis on renouvelle 30 cycles. Les dénaturations se font pendant 45 secondes à 95°C, l'hybridation pendant 30 secondes à 60°C et l'élongation pendant 1 minute à 72°C. Enfin, un cycle supplémentaire est mené avec une élongation de 10 minutes à 72°C.

### 1.2.3. Résultats.

Les résultats sont lus par migration sur gel d'agarose à 0,8% de 1/10 du produit de la réaction de PCR. En présence de bromure de 3,8-diamino-5-éthyl-6-phénylphénanthridinium (bromure d'éthydium), on visualise sur le gel une bande unique, intense, de taille légèrement supérieure au marqueur de poids moléculaire 300.

### 1.3. Sous clonage du produit PCR dans un vecteur permettant le séquençage.

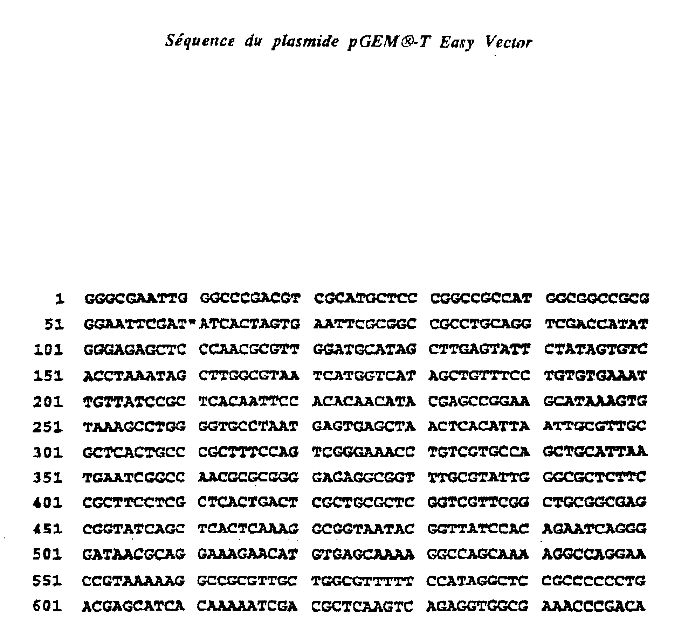

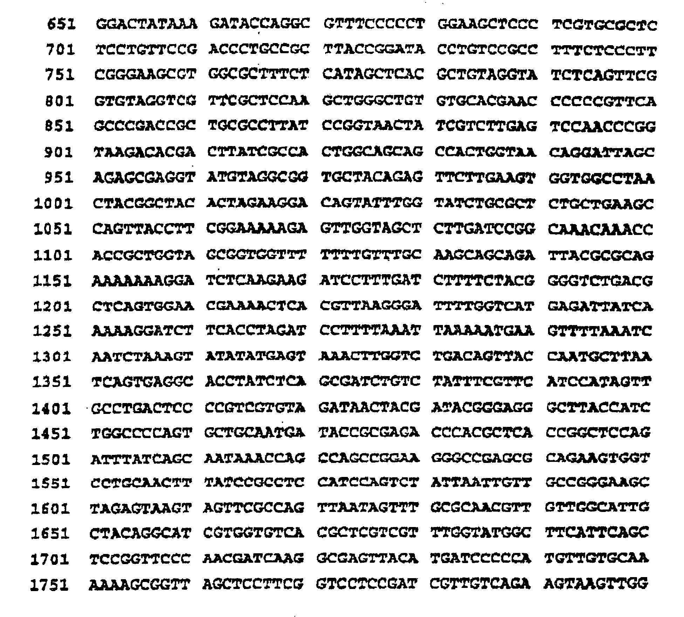

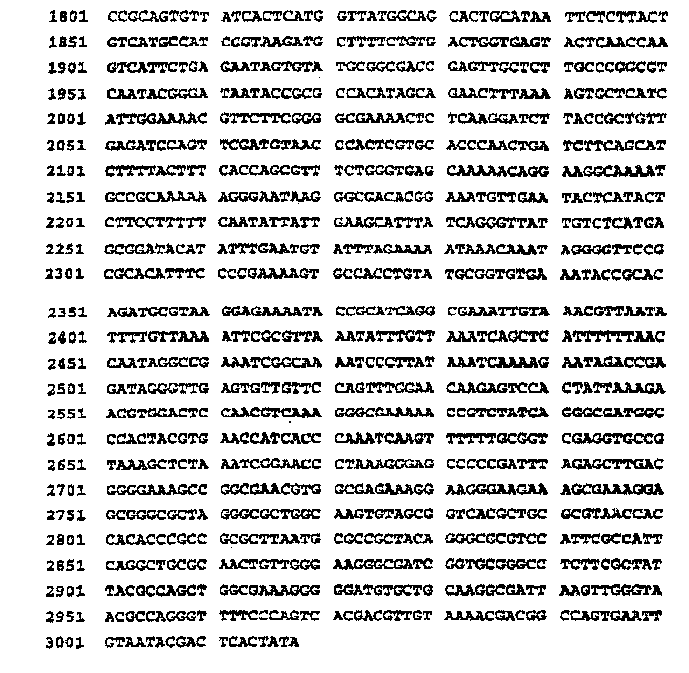

Le vecteur utilisé est le pGEM T-easy Vector (commercialisé par PROGEMA Corporation, Madison, U.S.A., réf. A 1380 - séquence donnée figures 1/3 - 3/3). Le plasmide pGEM®-T Easy Vector, a été linéarisé avec *Eco*R V à la base 60 de cette séquence (indiquée par une astérisque). On lui a ajouté un T aux deux extrémités 3'. Le T ajouté n'est pas inclus dans cette séquence. La séquence reproduite ci-après correspond à l'ARN synthétisé par la T7 ARN polymérase et est le complémentaire de l'ARN synthétisé par la SP6 ARN polymérase. Les étapes sont les suivantes :
- purification de la bande correspondante au produit PCR par électroélution,
- ligation durant une nuit à 16°C avec 1 µl de vecteur pGEM T-easy à 50 ng/µl, 1 µl de tampon ligase concentré 10 fois,
- 3 µl de produit extrait de la bande purifié estimée à 20 ng/µl,
- on complète avec de l'eau jusqu'à 10 µl.

Les bactéries JM 109 (génotype : e14⁻(McrA⁻) recA1 endA1 gyrA96 thi-1 hsdR17(r_{K-}m_{K+}) supE44 relA1 Δ(lac-proAB) [F' traD36 proAB lacI^{q}ZΔM15] - cf. Yanish-Perron, C., Viera, J., Messing, J., *Gene*, **33,** 103-199 (1985)) sont rendues compétentes par un traitement préalable au CaCl₂ puis transformées par un choc thermique de 45 secondes à 42°C avec 1/5 de la ligation. Les cellules sont ensuite mises en culture sur le milieu LB-ampicilline en boîte de Pétri pendant toute une nuit à 37°C.

On prépare l'ADN plasmidique de quelques clones recombinant. Puis le sous clonage est vérifié par la digestion enzymatique avec Eco RI.

### 1.4. Séquençage.

Il est effectué par la technique classique des didésoxynucléotides de SANGER sur le vecteur pGEM T-easy Vector ayant incorporé le produit PCR de 315 paires de bases (préparé à grande échelle par le kit QIAGEN tip 100). L'amorce utilisée pour le séquençage est l'oligonucléotide universel T7 présent sur le plasmide pGEM T-easy Vector.

### 1.5. Résultat.

Le séquence brute suivante est obtenue :

GTG TGT CTG TGC GTG GTG ATG GCA GTC CTG GCA GCA GGC GCC CTG
GCG CAG CCG GTA GTC CCT GTA GAA GCT GTG GAC CCT ATG GAG CAG
CGG GCG GAG GAG GCG CCC CGA AGG CAG CTG AGG GCT GTG CTC CGA
CCG GAC AGC GAG CCC CGA GCG CGC CTG GGC GCA CTG CTA GCC CGA
TAC ATC CAG CAG GTC CGC AAA GCT CCC TCT GGC CGC ATG TCC GTT
CTT AAG AAC CTG CAG GGC CTG GAC CCT AGC CAC AGG ATA AGT GAC
CGG GAC TAC ATG GGC TGG ATG GAT TTC GGC CGG CGC AGT GCT GAG

La traduction en acides aminés de la séquence obtenue aboutit à :

| | | | |
|---|---|---|---|
| VCLCVV | MAVLAAGALA | QPVVPVEAVD | PMEQRAEEAP |
| RRQLRAVLRP | DSEPRARLGA | LLARYIQQVR | KAPSGRMSVL |
| KNLQGLDPSH | RISDRDYMGW | MDFGRRSAE | |

permet bien de retrouver la séquence nucléotidique du précurseur de la CCK (dont la séquence a été fournie par la banque de données Swiss Prot n° p01355).

L'abréviation des acides aminés est la suivante :
- Alanine: A
- Argine: R
- Acide aspartique: D
- Asparagine: N
- Cystéine: C
- Acide glutamique: E
- Glutamine: Q
- Glycine: G
- Histidine: H
- Isoleucine: I
- Leucine: L
- Lysine: K
- Méthionine: M
- Phénylalanine: F
- Proline: P
- Sérine: S
- Thréonine: T
- Tryptophane: W
- Tyrosine: Y
- Valine: V

## Revendications

1. Oligonucléotide OX simple brin, **caractérisé en ce qu'**il comprend de 9 à 42 nucléotides et est capable de s'hybrider dans des conditions douces avec un oligonucléotide OY de séquence Y1-Y2-Y3-Y4-Y5, dans laquelle Y1 représente une séquence nucléotidique de 1 à 12 nucléotides ou Y1 est supprimé, Y2 représente un trinucléotide codant pour Gly, Y3 et Y4 représentent indépendamment un trinucléotide codant pour Arg ou Lys, et Y5 représente une séquence nucléotidique de 1 à 21 nucléotides ou Y5 est supprimé.

2. Oligonucléotide OX selon la revendication 1, **caractérisé en ce qu'**il comprend de 9 à 42 nucléotides et est capable de s'hybrider dans des conditions stringentes avec un oligonucléotide OY de séquence Y1-Y2-Y3-Y4-Y5, dans laquelle Y1 représente une séquence nucléotidique de 1 à 12 nucléotides ou Y1 est supprimé, Y2 représente un trinucléotide codant pour Gly, Y3 et Y4 représentent indépendamment un trinucléotide codant pour Arg ou Lys, et Y5 représente une séquence nucléotidique de 1 à 21 nucléotides ou Y5 est supprimé.

3. Oligonucléotide OX selon la revendication 1 ou 2, **caractérisé en ce que** Y1 est supprimé dans l'oligonucléotide OY.

4. Oligonucléotide OX selon la revendication 1, 2 ou 3, **caractérisé en ce que** Y5 est supprimé dans l'oligonucléotide OY.

5. Oligonucléotide OX selon la revendication 1, 2 ou 3, **caractérisé en ce que**, dans OY, Y5 représente une séquence nucléotidique Y6-Y7-Y8-Y9, dans laquelle Y6 représente un trinucléotide codant pour Ser, Thr, ou Tyr, Y7 représente un trinucléotide codant pour un acide aminé quelconque, Y8 représente un trinucléotide codant pour Glu ou Asp et Y9 représente une séquence nucléotidique comprenant de 1 à 12 nucléotides.

6. Oligonucléotide OX selon la revendication 5, **caractérisé en ce que** Y1 et Y9 sont supprimés dans l'oligonucléotide OY.

7. Oligonucléotide OX selon la revendication 6, **caractérisé en ce qu'**il peut s'hybrider avec ledit oligonucléotide OY dans lequel Y2 représente un trinucléotide codant pour Gly, Y3 représente un trinucléotide codant pour Lys, Y4 un trinucléotide codant pour Arg et Y5 une séquence de 3 trinucléotides codant pour Ser-Ala-Glu.

8. Oligonucléotide simple brin OY, **caractérisé en ce qu'**il comprend de 9 à 42 nucléotides de séquence Y1-Y2-Y3-Y4-Y5, dans laquelle Y1 représente une séquence nucléotidique de 1 à 12 nucléotides ou Y1 est supprimé, Y2 représente un trinucléotide codant pour Gly, Y3 et Y4 représentent indépendamment un trinucléotide codant pour Arg ou Lys, et Y5 représente une séquence nucléotidique de 1 à 21 nucléotides ou Y5 est supprimé.

9. Oligonucléotide OY selon la revendication 8, **caractérisé en ce que** Y1 est supprimé

10. Oligonucléotide OY selon la revendication 8 ou 9, **caractérisé en ce que** Y5 est supprimé.

11. Oligonucléotide OY selon la revendication 8 ou 9, **caractérisé en ce que** Y5 représente une séquence nucléotidique Y6-Y7-Y8-Y9, dans laquelle Y6 représente un trinucléotide codant pour Ser, Thr, ou Tyr, Y7 représente un trinucléotide codant pour un acide aminé quelconque, Y8 représente un trinucléotide codant pour Glu ou Asp et Y9 représente une séquence nucléotidique comprenant de 1 à 12 nucléotides.

12. Oligonucléotide OY selon la revendication 11, **caractérisé en ce que** Y 1 et Y9 sont supprimés.

13. Oligonucléotide OY selon la revendication 12, **caractérisé en ce que** Y2 représente un trinucléotide codant pour Gly, Y3 représente un trinucléotide codant pour Lys, Y4 un trinucléotide codant pour Arg et Y5 une séquence de 3 trinucléotides codant pour Ser-Ala-Glu.

14. Oligonucléotide simple brin OZ, **caractérisé en ce qu'**il comprend de 15 à 39 nucléotides et est capable de s'hybrider en conditions douces ou stringentes avec une séquence consensus signal caractéristique des hormones polypeptidiques amidées, ladite séquence a pour formule Z1-Z2-Z3-Z4-Z5-Z6-Z7 dans laquelle Z1 représente une séquence nucléotidique de 1 à 12 nucléotides ou Z1 est supprimé, Z2 et Z3 représentent deux trinucléotides codant pour Leu, Z4 et Z5 représentent deux trinucléotides codant pour deux acides aminés quelconques, Z6 représente un trinucléotide codant pour Leu, et Z7 représente une séquence nucléotidique de 1 à 12 nucléotides ou Z7 est supprimé.

15. Ensemble d'oligonucléotides OX selon l'une quelconque des revendications 1 à 7 ou d'oligonucléotides OZ selon la revendication 14, **caractérisé en ce qu'**il constitue une librairie combinatoire.

16. Méthode d'identification du précurseur d'un peptide ayant une extrémité C-terminale amidée, **caractérisée par** les étapes successives suivantes :
- obtention d'une banque d'ADN:
- hybridation d'un ou plusieurs oligonucléotides selon l'une quelconque des revendications 1 à 7 avec ladite banque d'ADN;
- identification de la ou les séquences d'ADN de ladite banque qui s'hybride avec un oligonucléotide selon l'une quelconque des revendications 1 à 7;
- identification dans cette ou ces séquences d'un ou plusieurs précurseurs de peptides avec une possible extrémité C-terminal amidée.

17. Méthode selon la revendication 16, **caractérisée en ce que** l'étape d'hybridation utilise une librairie combinatoire selon la revendication 15.

18. Méthode d'identification du précurseur d'un peptide ayant une extrémité C-terminale amidée, **caractérisée par** les étapes successives suivantes :
- obtention d'une banque d'ADN:
- utilisation de la technique PCR pour amplifier le fragment d'intérêt à l'aide d'un ensemble d'oligonucléotides selon l'une quelconque des revendications 1 à 7 et d'un autre ensemble d'oligonucléotides selon la revendication 14;
- identification de la ou les séquences d'ADN de ladite banque qui s'hybride avec un oligonucléotide selon l'une quelconque des revendications 1 à 7;
- identification dans cette ou ces séquences d'un ou plusieurs précurseurs de peptides avec une possible extrémité C-terminal amidée.

19. Méthode selon la revendication 18, **caractérisée en ce que** l'étape d'amplification utilise une librairie combinatoire selon la revendication 15.

20. Méthode d'identification du précurseur d'un peptide ayant une extrémité C-terminale amidée, **caractérisée par** les étapes successives suivantes :
- obtention d'une banque d'ADN;
- utilisation de la technique PCR pour amplifier le fragment d'intérêt à l'aide d'un ensemble d'oligonucléotides selon l'une quelconque des revendications 1 à 7;
- identification de la ou les séquences d'ADN de ladite banque qui s'hybride avec un oligonucléotide selon l'une quelconque des revendications 1 à 7;
- identification dans cette ou ces séquences d'un ou plusieurs précurseurs de peptides avec une possible extrémité C-terminal amidée.

21. Méthode selon la revendication 20, **caractérisée en ce que** l'étape d'amplification utilise une librairie combinatoire selon la revendication 15.

22. Méthode d'identification du précurseur d'un polypeptide ayant une extrémité C-terminale amidée, **caractérisée par** les étapes suivantes :
- obtention d'une banque d'ADN;
- utilisation de la technique PCR pour amplifier le fragment d'intérêt à l'aide d'un oligonucléotide selon l'une quelconque des revendication 1 à 7 et un autre oligonucléotide simple brin capable de s'hybrider en conditions douces ou stringentes avec une séquence consensus universelle contenue dans la séquence du vecteur plasmidique dans lequel sont clonés les ADNc de la dite banque d'ADN, tels que les primers T3, T7, KS, SK, M13, Reverse;
- identification de la séquence d'ADNc de ladite banque qui s'hybride avec un oligonucléotide selon l'une quelconque des revendication 1 à 7;
- identification dans cette séquence d'un ou plusieurs précurseurs de peptides avec une possible extrémité C-terminal amidée.

23. Méthode selon la revendication 22, **caractérisée en ce que** l'étape d'amplification utilise une librairie combinatoire selon la revendication 15.

24. Méthode selon l'une quelconque des revendications 16 à 23, **caractérisée en ce que** la banque d'ADN est une banque d'ADNc.

25. Méthode selon l'une quelconque des revendications 16 à 24, **caractérisée en ce que** l'oligonucléotide simple brin est détectable à l'aide d'un agent de marquage, tel que le ³²P ou la digoxygénine.

## Patentansprüche

1. Einzelsträngiges Oligonukleotid OX, **dadurch gekennzeichnet, daß** es 9 bis 42 Nukleotide umfaßt und bei milden Bedingungen mit einem Oligonukleotid OY der Sequenz Y1-Y2-Y3-Y4-Y5 hybridisieren kann, in der Y1 eine Nukleotidsequenz von 1 bis 12 Nukleotiden darstellt oder Y1 unterdrückt ist, Y2 ein für Gly kodierendes Trinukleotid darstellt, Y3 und Y4 unabhängig voneinander ein für Arg oder Lys kodierendes Trinukleotid darstellen und Y5 eine Nukleotidsequenz von 1 bis 21 Nukleotiden darstellt oder Y5 unterdrückt ist.

2. Oligonukleotid OX nach Anspruch 1, **dadurch gekennzeichnet, daß** es 9 bis 42 Nukleotide umfaßt und bei stringenten Bedingungen mit einem Oligonukleotid OY der Sequenz Y1-Y2-Y3-Y4-Y5 hybridisieren kann, in der Y1 eine Nukleotidsequenz von 1 bis 12 Nukleotiden darstellt oder Y1 unterdrückt ist, Y2 ein für Gly kodierendes Trinukleotid darstellt, Y3 und Y4 unabhängig voneinander ein für Arg oder Lys kodierendes Trinukleotid darstellen und Y5 eine Nukleotidsequenz von 1 bis 21 Nukleotiden darstellt oder Y5 unterdrückt ist.

3. Oligonukleotid OX nach Anspruch 1 oder 2, **dadurch gekennzeichnet, daß** Y1 in dem Oligonukleotid OY unterdrückt ist.

4. Oligonukleotid OX nach Anspruch 1, 2 oder 3, **dadurch gekennzeichnet, daß** Y5 in dem Oligonukleotid OY unterdrückt ist.

5. Oligonukleotid OX nach Anspruch 1, 2 oder 3, **dadurch gekennzeichnet, daß** Y5 in OY eine Nukleotidsequenz Y6-Y7-Y8-Y9 darstellt, in der Y6 ein für Ser, Thr oder Tyr kodierendes Trinukleotid darstellt, Y7 ein für eine beliebige Aminosäure kodierendes Trinukleotid darstellt, Y8 ein für Glu oder Asp kodierendes Trinukleotid darstellt und Y9 eine Nukleotidsequenz mit 1 bis 12 Nukleotiden darstellt.

6. Oligonukleotid OX nach Anspruch 5, **dadurch gekennzeichnet, daß** Y1 und Y9 in dem Oligonukleotid OY unterdrückt sind.

7. Oligonukleotid OX nach Anspruch 6, **dadurch gekennzeichnet, daß** es mit dem Oligonukleotid OY hybridisieren kann, in dem Y2 ein für Gly kodierendes Trinukleotid darstellt, Y3 ein für Lys kodierendes Trinukleotid darstellt, Y4 ein für Arg kodierendes Trinukleotid und Y5 eine Sequenz von 3 für Ser-Ala-Glu kodierenden Trinukleotiden darstellt.

8. Einzelsträngiges Oligonukleotid OY, **dadurch gekennzeichnet, daß** es 9 bis 42 Nukleotide der Sequenz Y1-Y2-Y3-Y4-Y5 umfaßt, in der Y1 eine Nukleotidsequenz von 1 bis 12 Nukleotiden darstellt oder Y1 unterdrückt ist, Y2 ein für Gly kodierendes Trinukleotid darstellt, Y3 und Y4 unabhängig voneinander ein für Arg oder Lys kodierendes Trinukleotid darstellen und Y5 eine Nukleotidsequenz von 1 bis 21 Nukleotiden darstellt oder Y5 unterdrückt ist.

9. Oligonukleotid OY nach Anspruch 8, **dadurch gekennzeichnet, daß** Y1 unterdrückt ist.

10. Oligonukleotid OY nach Anspruch 8 oder 9, **dadurch gekennzeichnet, daß** Y5 unterdrückt ist.

11. Oligonukleotid OY nach Anspruch 8 oder 9, **dadurch gekennzeichnet, daß** Y5 eine Nukleotidsequenz Y6-Y7-Y8-Y9 darstellt, in der Y6 ein für Ser, Thr oder Tyr kodierendes Trinukleotid darstellt, Y7 ein für eine beliebige Aminosäure kodierendes Trinukleotid darstellt, Y8 ein für Glu oder Asp kodierendes Trinukleotid darstellt und Y9 eine Nukleotidsequenz mit 1 bis 12 Nukleotiden darstellt.

12. Oligonukleotid OY nach Anspruch 11, **dadurch gekennzeichnet, daß** Y1 und Y9 unterdrückt sind.

13. Oligonukleotid OY nach Anspruch 12, **dadurch gekennzeichnet, daß** Y2 ein für Gly kodierendes Trinukleotid darstellt, Y3 ein für Lys kodierendes Trinukleotid darstellt, Y4 ein für Arg kodierendes Trinukleotid darstellt und Y5 eine Sequenz von 3 für Ser-Ala-Glu kodierenden Trinukleotiden darstellt.

14. Einzelsträngiges Oligonukleotid OZ, **dadurch gekennzeichnet, daß** es 15 bis 39 Nukleotide umfaßt und bei milden oder stringenten Bedingungen mit einer für die amidierten Polypeptidhormone charakteristischen Konsensus-Signal-Sequenz hybridisieren können, die die Formel Z1-Z2-Z3-Z4-Z5-Z6-Z7 hat, in der Z1 eine Nukleotidsequenz von 1 bis 12 Nukleotiden darstellt oder Z1 unterdrückt ist, Z2 und Z3 zwei für Leu kodierende Trinukleotiden darstellen, Z4 und Z5 zwei für zwei beliebige Aminosäuren kodierende Trinukleotide darstellen, Z6 ein für Leu kodierendes Trinukleotid darstellt und Z7 eine Nukleotidsequenz von 1 bis 12 Nukleotiden darstellt oder Z7 unterdrückt ist.

15. Gruppe von Oligonukleotiden OX nach einem der Ansprüche 1 bis 7 oder von Oligonukleotiden OZ nach Anspruch 14, **dadurch gekennzeichnet, daß** sie eine kombinatorische Bibliothek bildet.

16. Verfahren zur Identifizierung eines Vorläufers eines Peptids mit einem endständigen amidierten C-Atom, **gekennzeichnet durch** die folgenden aufeinanderfolgenden Schritte:
- Erstellung einer DNA-Bank;
- Hybridisierung einer oder mehrerer Oligonukleotide nach einem der Ansprüche 1 bis 7 mit der DNA-Bank;
- Identifizierung der DNA-Sequenz(en) der Bank, die mit einem Oligonukleotid nach einem der Ansprüche 1 bis 7 hybridisiert (en) ;
- Identifizierung dieser Sequenz(en) von einem oder mehreren Peptidvorläufern mit einem möglichen endständigen amidierten C-Atom.

17. Verfahren nach Anspruch 16, **dadurch gekennzeichnet, daß** im Schritt der Hybridisierung eine kombinatorische Bibliothek nach Anspruch 15 verwendet wird.

18. Verfahren zur Identifizierung des Vorläufers eines Peptids mit einem endständigen amidierten C-Atom, **gekennzeichnet durch** die folgenden aufeinanderfolgenden Schritte:
- Erstellung einer DNA-Bank;
- Verwendung der PCR-Technik zur Vervielfältigung des Fragments von Interesse mit Hilfe einer Gruppe von Oligonukleotiden nach einem der Ansprüche 1 bis 7 und einer anderen Gruppe von Oligonukleotiden nach Anspruch 14;
- Identifizierung der DNA-Sequenz(en) der Bank, die mit einem Oligonukleotid nach einem der Ansprüche 1 bis 7 hybridisiert(en);
- Identifizierung eines oder mehrerer Peptidvorläufer mit einem möglichen endständigen amidierten C-Atom in dieser Sequenz oder in diesen Sequenzen.

19. Verfahren nach Anspruch 18, **dadurch gekennzeichnet, daß** in dem Schritt der Vervielfältigung eine kombinatorische Bibliothek nach Anspruch 15 verwendet wird.

20. Verfahren zur Identifizierung eines Vorläufers eines Peptids mit einem endständigen amidierten C-Atom, **gekennzeichnet durch** die folgenden aufeinanderfolgenden Schritte:
- Erstellung einer DNA-Bank;
- Verwendung der PCR-Technik zur Vervielfältigung des Fragments von Interesse mit Hilfe einer Gruppe von Oligonukleotiden nach einem der Ansprüche 1 bis 7:
- Identifizierung der DNA-Sequenz(en) der Bank, die mit einem Oligonukleotid nach einem der Ansprüche 1 bis 7 hybridisiert(en),
- Identifizierung eines oder mehrerer Peptidvorläufer mit einem möglichen endständigen amidierten C-Atom in dieser Sequenz oder in diesen Sequenzen.

21. Verfahren nach Anspruch 20, **dadurch gekennzeichnet, daß** im Schritt der Vervielfältigung eine kombinatorische Bibliothek nach Anspruch 15 verwendet wird.

22. Verfahren zur Identifizierung des Vorläufers eines Polypeptids mit einem endständigen amidierten C-Atom, **gekennzeichnet durch** die folgenden aufeinanderfolgenden Schritte:
- Herstellung einer DNA-Bank;
- Verwendung der PCR-Technik zur Vervielfältigung des Fragments von Interesse mit Hilfe eines Oligonukleotids nach einem der Ansprüche 1 bis 7 und einem anderen einsträngigen Oligonukleotid, das bei milden oder stringenten Bedingungen mit einer universellen Konsensus-Sequenz hybridisieren kann, die in der Sequenz des Plasmidvektors enthalten ist, in dem die cDNA der DNA-Bank geklont sind, wie z.B. die Primer T3, T7, KS, SK, M13, Revers;
- Identifizierung der cDNA-Sequenz der Bank, die mit einem Oligonukleotid nach einem der Ansprüche 1 bis 7 hybridisiert;
- Identifizierung eines oder mehrerer Peptidvorläufer mit einem möglichen endständigen amidierten C-Atom in dieser Sequenz.

23. Verfahren nach Anspruch 22, **dadurch gekennzeichnet, daß** im Schritt der Vervielfältigung eine kombinatorische Bibliothek nach Anspruch 15 verwendet wird.

24. Verfahren nach einem der Ansprüche 16 bis 24, **dadurch gekennzeichnet, daß** die DNA-Bank eine cDNA-Bank ist.

25. Verfahren nach einem der Ansprüche 16 bis 24, **dadurch gekennzeichnet, daß** das einzelsträngige Oligonukleotid mit Hilfe eines Markiermittels, wie z.B. ³²P oder Digoxygenin erfaßbar ist.

## Claims

1. Single-stranded oligonucleotide OX, **characterized in that** it comprises 9 to 42 nucleotides and is capable of hybridizing under mild conditions with an oligonucleotide OY of the sequence Y1-Y2-Y3-Y4-Y5, in which Y1 represents a nucleotide sequence of 1 to 12 nucleotides or Y1 is suppressed, Y2 represents a trinucleotide which codes for Gly, Y3 and Y4 independently represent a trinucleotide which codes for Arg or Lys and Y5 represents a nucleotide sequence of 1 to 21 nucleotides or Y5 is suppressed.

2. Oligonucleotide OX according to claim 1, **characterized in that** it comprises 9 to 42 nucleotides and is capable of hybridizing under stringent conditions with an oligonucleotide OY of the sequence Y1-Y2-Y3-Y4-Y5, in which Y1 represents a nucleotide sequence of 1 to 12 nucleotides or Y1 is suppressed, Y2 represents a trinucleotide which codes for Gly, Y3 and Y4 independently represent a trinucleotide which codes for Arg or Lys and Y5 represents a nucleotide sequence of 1 to 21 nucleotides or Y5 is suppressed.

3. Oligonucleotide OX according to claim 1 or 2, **characterized in that** Y1 is suppressed in the oligonucleotide OY.

4. Oligonucleotide OX according to claim 1, 2 or 3, **characterized in that** Y5 is suppressed in the oligonucleotide OY.

5. Oligonucleotide OX according to claim 1, 2 or 3, **characterized in that**, in OY, Y5 represents a nucleotide sequence Y6-Y7-Y8-Y9, in which Y6 represents a trinucleotide which codes for Ser, Thr or Tyr, Y7 represents a trinucleotide which codes for any amino acid, Y8 represents a trinucleotide which codes for Glu or Asp and Y9 represents a nucleotide sequence comprising 1 to 12 nucleotides.

6. Oligonucleotide OX according to claim 5, **characterized in that** Y1 and Y9 are suppressed in the oligonucleotide OY.

7. Oligonucleotide OX according to claim 6, **characterized in that** it can hybridize with the said oligonucleotide OY in which Y2 represents a trinucleotide which codes for Gly, Y3 represents a trinucleotide which codes for Lys, Y4 represents a trinucleotide which codes for Arg and Y5 represents a sequence of 3 trinucleotides which codes for Ser-Ala-Glu.

8. Single-stranded oligonucleotide OY, **characterized in that** it comprises 9 to 42 nucleotides of the sequence Y1-Y2-Y3-Y4-Y5, in which Y1 represents a nucleotide sequence of 1 to 12 nucleotides or Y1 is suppressed, Y2 represents a trinucleotide which codes for Gly, Y3 and Y4 independently represent a trinucleotide which codes for Arg or Lys and Y5 represents a nucleotide sequence of 1 to 21 nucleotides or Y5 is suppressed.

9. Oligonucleotide OY according to claim 8, **characterized in that** Y1 is suppressed.

10. Oligonucleotide OY according to claim 8 or 9, **characterized in that** Y5 is suppressed.

11. Oligonucleotide OY according to claim 8 or 9, **characterized in that** Y5 represents a nucleotide sequence Y6-Y7-Y8-Y9, in which Y6 represents a trinucleotide which codes for Ser, Thr or Tyr, Y7 represents a trinucleotide which codes for any amino acid, Y8 represents a trinucleotide which codes for Glu or Asp and Y9 represents a nucleotide sequence comprising I to 12 nucleotides.

12. Oligonucleotide OY according to claim 11, **characterized in that** Y1 and Y9 are suppressed.

13. Oligonucleotide OY according to claim 12, **characterized in that** Y2 represents a trinucleotide which codes for Gly, Y3 represents a trinucleotide which codes for Lys, Y4 represents a trinucleotide which codes for Arg and Y5 represents a sequence of 3 trinucleotides which codes for Ser-Ala-Glu.

14. Single-stranded oligonucleotide OZ, **characterized in that** it comprises 15 to 39 nucleotides and is capable of hybridizing under mild or stringent conditions with a consensus signal sequence characteristic of amidated polypeptide hormones, the said sequence having Z1-Z2-Z3-Z4-Z5-Z6-Z7 as formula, in which Z1 represents a nucleotide sequence of 1 to 12 nucleotides or Z1 is suppressed, Z2 and Z3 represent two trinucleotides which code for Leu, Z4 and Z5 represent two trinucleotides which code for any two amino acids, Z6 represents a trinucleotide which codes for Leu and Z7 represents a nucleotide sequence of 1 to 12 nucleotides or Z7 is suppressed.

15. Group of oligonucleotides OX according to any one of claims 1 to 7 or of oligonucleotides OZ according to claim 14, **characterized in that** it constitutes a combinatorial library.

16. Method for identification of the precursor of a peptide having an amidated C-terminal end, **characterized by** the following successive stages:
- obtaining of a DNA bank;
- hybridization of one or more oligonucleotides according to any one of claims 1 to 7 with the said DNA bank;
- identification of the DNA sequence or sequences of the said bank which hybridizes with an oligonucleotide according to any one of claims 1 to 7;
- identification in this sequence or sequences of one or more peptide precursors with a possible amidated C-terminal end.

17. Method according to claim 16, **characterized in that** the hybridization stage uses a combinatorial library according to claim 15.

18. Method for identification of the precursor of a peptide having an amidated C-terminal end, **characterized by** the following successive stages:
- obtaining of a DNA bank;
- use of the PCR technique to amplify the fragment of interest with the aid of a group of oligonucleotides according to any one of claims 1 to 7 and another group of oligonucleotides according to claim 14;
- identification of the DNA sequence or sequences of the said bank which hybridizes with an oligonucleotide according to any one of claims 1 to 7;
- identification in this sequence or sequences of one or more peptide precursors with a possible amidated C-terminal end.

19. Method according to claim 18, **characterized in that** the amplification stage uses a combinatorial library according to claim 15.

20. Method for identification of the precursor of a peptide having an amidated C-terminal end, **characterized by** the following successive stages:
- obtaining of a DNA bank;
- use of the PCR technique to amplify the fragment of interest with the aid of a group of oligonucleotides according to any one of claims 1 to 7;
- identification of the DNA sequence or sequences of the said bank which hybridizes with an oligonucleotide according to any one of claims 1 to 7;
- identification in this sequence or sequences of one or more peptide precursors with a possible amidated C-terminal end.

21. Method according to claim 20, **characterized in that** the amplification stage uses a combinatorial library according to claim 15.

22. Method for identification of the precursor of a polypeptide having an amidated C-terminal end, **characterized by** the following stages:
- obtaining of a DNA bank;
- use of the PCR technique to amplify the fragment of interest with the aid of an oligonucleotide according to any one of claims 1 to 7 and another single-stranded oligonucleotide capable of hybridizing under mild or stringent conditions with a universal consensus sequence contained in the sequence of the plasmid vector in which the cDNA of the said DNA bank are cloned, such as the primers T3, T7, KS, SK, M13, Reverse;
- identification of the cDNA sequence of the said bank which hybridizes with an oligonucleotide according to any one of claims 1 to 7;
- identification in this sequence of one or more peptides with a possible amidated C-terminal end.

23. Method according to claim 22, **characterized in that** the amplification stage uses a combinatorial library according to claim 15.

24. Method according to any one of claims 16 to 23, **characterized in that** the DNA bank is cDNA bank.

25. Method according to any one of claims 16 to 24, **characterized in that** the single-stranded oligonucleotide can be detected with the aid of a marking agent, such as ³²P or digoxigenin.
